# EUROPÄISCHE PATENTSCHRIFT

(11) **EP 1 558 309 B1**
(45) Veröffentlichungstag und Bekanntmachung des Hinweises auf die Patenterteilung: **17.02.2010**
(21) Anmeldenummer: 03808241.8
(22) Anmeldetag: 09.10.2003
(51) Int. Cl.: A61M 5/315

(54) **INJEKTIONSVORRICHTUNG MIT LEERHUB**
INJECTION DEVICE WITH BACKLASH
DISPOSITIF D'INJECTION AVEC COURSE A VIDE

(30) Priorität: 15.10.2002 DE 10248061
(43) Veröffentlichungstag der Anmeldung: 03.08.2005
(73) Patentinhaber: TecPharma Licensing AG, 3401 Burgdorf (CH)
(72) Erfinder: KIRCHHOFER, Fritz, CH-3454 Sumiswald (CH); BUCHER, Eugen, CH-3127 Muehlethurn (CH)
(86) Internationale Anmeldenummer: PCT/CH2003/000663
(87) Internationale Veröffentlichungsnummer: WO 2004/035117

(56) Entgegenhaltungen:
- EP-A- 0 594 357
- WO-A-90/09202
- WO-A-98/39041
- WO-A-03/020347
- DE-A- 3 814 023
- DE-A- 3 900 926
- US-A- 4 883 472

## Beschreibung

Die Erfindung betrifft eine Vorrichtung zur dosierten Verabreichung eines injizierbaren Produkts, insbesondere einen Injektionspen z.B. zur Verabreichung von Insulin.

Eine Vorrichtung, wie die Erfindung sie betrifft, ist beispielsweise aus der WO 97/36626 bekannt. Die Vorrichtung weist ein Gehäuse mit einem Reservoir für das Produkt auf. In dem Reservoir ist ein Kolben aufgenommen, der bei einer Verschiebung in eine Vorschubrichtung das Produkt aus dem Reservoir durch einen Auslass des Reservoirs verdrängt. Eine Zahnstange wirkt als Kolbenstange und schiebt den Kolben in Vorschubrichtung. Im Gehäuse ist ferner ein Antriebsglied relativ zum Gehäuse in und gegen die Vorschubrichtung verschiebbar aufgenommen, das bei einer Verschiebung in Vorschubrichtung die Zahnstange mitnimmt. Hierfür greift das Antriebsglied mit Mitnehmern in Zahnreihen der Zahnstange ein. Zum Einstellen derjenigen Produktmenge, die mit einem Hub verabreicht wird, d.h. durch die Betätigung einer Dosiereinrichtung wird das Antriebsglied in einer vorderen Stellung um eine eingestellte Dosisweglänge manuell gegen die Vorschubrichtung zurückgezogen. Dabei gleiten die Mitnehmer des Antriebsgliedes, über die Zähne der Zahnreihen der Zahnstange und geben dabei elastisch nach. Ein Zurückverschieben der Zahnstange wird durch relativ zum Gehäuse verschiebegesichert aufgenommene Sperrmittel verhindert. Die Sperrmittel wirken mit einer der Zahnreihen der Zahnstange derart zusammen, dass die Sperrmittel eine Verschiebung der Zahnstange gegen die Vorschubrichtung verhindern. Durch elastisches Nachgeben erlauben sie eine Verschiebung der Zahnstange in Vorschubrichtung. Durch die Betätigung des Antriebsknopfes wird mittels des Antriebsglieds die eingestellte Dosisweglänge von der Zahnstange, bzw. dem Kolben, zurückgelegt, sodass die eingestellte Dosis durch den Auslass des Reservoirs ausgeschüttet wird.

Die EP 0498737 offenbart eine Dosierungseinrichtung für ein Injektionsgerät, das einen Mechanismus zur Dosiseinstellung und zur Verabreichung des Produkts aufweist. Bei dem Mechanismus wird eine Hülse zwischen zwei Einstellungen bewegt. In der Stellung für die Dosiseinstellung wird die Vorschubweglänge für eine Kolbenstange und damit die Dosismenge eingestellt. Dabei befinden sich Eingriffsbacken, die mit einem Antriebsglied in Verbindung stehen, nicht im Eingriff mit der Kolbenstange. Durch das Drehen der Hülse in die zweite Stellung gelangt das Injektionsgerät in einen injektionsbereiten zweiten Zustand. Dabei greifen die Backen zwischen Zähne einer Zahnreihe der Kolbenstange ein, indem das Antriebsglied gedrückt wird. Beim Eindrücken des Antriebsgliedes greifen die Backen unmittelbar in die Zahnreihe ein. Ferner weist das Injektionsgerät eine Rückzugssperre für die Kolbenstange auf Hierfür sind im Inneren des Injektionsgeräts zwei Krallen vorgesehen, die in entspanntem Zustand nicht in die Kolbenstange eingreifen. In diesem Zustand ist die Kolbenstange vor und zurück beweglich. Durch das Eindrehen einer Ampulle in das Injektionsgerät wirkt eine Kante, die innerhalb einer Aufnahmehülse für die Ampulle vorgesehen ist, gegen die Krallen und drückt deren Zähne in die Zahnreihe der Kolbenstange. Durch die Anordnung der Zähne der Zahnstange und der Krallen ist der Eingriff derart ausgebildet, dass eine Bewegung der Kolbenstange in Vorschubrichtung möglich ist, jedoch nicht in einer entgegengesetzten Richtung.

In der US 6,228,067 ist ein Injektionsgerät beschrieben, das ebenfalls eine Dosier- und Antriebseinrichtung aufweist. Durch das Verdrehen zweier Gehäusebereiche gegeneinander wird eine axiale Bewegung eines Dosierungselements in einem der Gehäuseteile ausgelöst, wodurch eine bestimmte Dosis eingestellt werden kann. Dabei steht die Antriebseinrichtung zunächst in Eingriff mit einer Zahnstange, welche den Kolben zur Dosisabgabe vorschiebt. Durch das Zurückziehen eines Betätigungsgliedes wird die Zahnstange entgegen der Vorschubrichtung zurückgezogen, wobei der Kolben in seiner Position verbleibt, indem Eingriffsvorsprünge zwischen Zähne einer Zahnreihe der Zahnstange eingreifen. Die Eingriffsvorsprünge sind an Verlängerungen eines Betätigungsknopfes angeordnet und sind, wenn sie sich im Eingriff befinden, vorgespannt. Durch das Herausziehen des Betätigungsknopfes wird die Kolbenstange so lange entgegen der Vorschubrichtung bewegt, bis Nocken, die an den Verlängerungen gegenüber den Vorsprüngen vorgesehen sind, aufgrund ihrer Vorspannung in Aussparungen der Gehäuseinnenfläche eingreifen. Dadurch werden die Vorsprünge aus den Zwischenräumen der Zahnreihe der Zahnstange gezogen, sodass diese freigegeben wird. Durch eine Feder wird sie in Vorschubrichtung bewegt, bis sie an dem Kolben anliegt. Zur Injektion wird der Betätigungsknopf in Vorschubrichtung bewegt, wobei die Nocken der Verlängerungen aus den Aussparungen in der Innenfläche des Gehäuses gedrückt werden und dabei die Vorsprünge zwischen die Zähne der Zahnreihe schieben, sodass der Betätigungsknopf wieder in Eingriff mit der Kolbenstange steht.

Die Dosisweglänge bei den Injektionsgeräten nach dem obengenannten Stand der Technik sind im allgemeinen sehr kurz, sodass zur Injektion der Betätigungsknopf ebenfalls nur um diese geringe Weglänge bewegt werden muss. Sobald der Betätigungsknopf in Vorschubrichtung bewegt wird, greifen die verschiedenen Antriebseinrichtungen an der Kolbenstange an und bewegen diese in Vorschubrichtung, sodass die gewünschte Dosis ausgeschüttet wird. Durch den geringen Bewegungsspielraum, der bei der Injektion für den Betätigungsknopf verbleibt, ist es für einen Anwender oft schwer erkennbar, in welcher Stellung, bzw. in welchem Zustand, sich das Injektionsgerät befindet. Um sicherzustellen, dass auch die gesamte Dosisweglänge bei der Injektion durchlaufen wurde, wird oft eine unnötig hohe Kraft auf den Betätigungsknopf oder das gesamte Injektionsgerät ausgeübt. Dadurch kann es zu Unsicherheiten der Führung des Gerätes oder auch zu übermäßigem Materialverschleiß kommen.

Die US4883472 beschreibt ein Injektionsgerät gemäß der Präambel des Anspruchs 1, wobei die elastische Konstante der ersten Feder mit der elastischen Konstante der zweiten Feder so abgestimmt ist, dass bei einer Injektion zuerst eine Leerhubstrecke, dann eine Dosisweglänge und zuletzt eine weiter Leerhubstrecke ausgeführt wird.

Die DE3900926C1 offenbart ein spritzenförmiges Injektionsgerät, wobei nach der Dosisabgabe die Abgabemechanik arretiert wird, sodass nach Durchführung der Injektion wieder eine definierte Anfangsposition erreicht wird.

Die EP594357A1 beschreibt ein Enddosisindikator, der ein Click-Geräusch erzeugt.

Es ist daher die Aufgabe der vorliegenden Erfmdung, eine alternative Vorrichtung zur dosierten Verabreichung eines injizierbaren Produktes vorzusehen, die eine einfache Handhabung und eine sichere Führung beim Einsatz der Verabreichungsvorrichtung und eine zuverlässige Ausschüttung einer gewünschten Dosis gewährleistet, insbesondere bei kleinen Dosisweglängen soll die Kontrolle der Verabreichungsvorrichtung spürbar und sichtbar verbessert werden.

Diese Aufgabe wird durch die kennzeichnenden Merkmale einer Verabreichungsvorrichtung gemäß dem Anspruch 1 erfüllt. Bevorzugte Ausgestaltungen gehen aus den Unteransprüchen hervor.

Die erfindungsgemäße Verabreichungsvorrichtung weist ein Gehäuse mit einem Reservoir für ein zu injizierendes Produkt auf. Dabei kann das Reservoir wieder befüllbar sein oder z.B. auch durch eine austauschbare Ampulle gebildet werden. Ein Kolben verdrängt bei einer Verschiebung in Vorschubrichtung das Produkt durch einen Auslass aus dem Reservoir. Zur Verschiebung des Kolbens dient wenigstens ein Antriebsglied, das z. B. durch eine Kolbenstange, vorzugsweise eine Zahnstange gegeben ist. Es ist auch möglich mehrere Antriebsglieder zu verwenden, wie etwa eine Zahnstange und ein Glied, das zum Vorschub in die Zahnstange eingreift. Die Kolbenstange wird durch die Betätigung eines Antriebsknopfes durch den Anwender in Vorschubrichtung verschoben. Vorzugsweise ragt der Antriebsknopf axial aus dem Ende des Gehäuses, das dem Produktauslass gegenüberliegt. Der Antriebsknopf kann direkt mit der Kolbenstange zusammenwirken oder auf ein weiteres Antriebsglied einwirken, das wiederum mit der Kolbenstange in Kontakt tritt.

Erfindungsgemäß weist die Verabreichungsvorrichtung wenigstens ein Verzögerungsglied auf, das aus einer ersten Stellung in eine zweite Stellung, die zur ersten Stellung beabstandet ist, relativ zu dem Antriebsglied, d. h. zu der Kolbenstange, durch die Betätigung des Antriebsknopfes in Längsrichtung der Vorrichtung verschiebbar ist. Die Kolbenstange und damit der Kolben, der auf das Produkt in dem Reservoir einwirkt, bleibt bei der Verschiebung des Verzögerungsglieds von der ersten in die zweite Stellung in Bezug zu dem Gehäuse, bzw. dem Reservoir, in Ruhe. Bei der Verschiebung des Verzögerungsglieds von der ersten in die zweite Stellung entsteht eine Leerhubstrecke für den Antriebsknopf, da trotz seiner Betätigung über diese Strecke kein Produkt verabreicht wird. Das Verzögerungsglied ist vorzugsweise fest mit dem Antriebsknopf verbunden oder mit diesem einstückig ausgebildet. Dabei kann es das Antriebsglied oder die Kolbenstange hülsenförmig umschließen oder lediglich eine balkenartige Verlängerung von dem Antriebsknopf darstellen. Die erste und die zweite Stellung des Verzögerungsgliedes sind bevorzugt derart lösbar feste Stellungen, dass sie ohne eine Krafteinwirkung, d.h. ohne eine Betätigung des Antriebsknopfes, in dieser bestimmten Position verbleiben. Erst durch Krafteinwirkung, d.h. durch die Betätigung des Antriebsknopfes, kann das Verzögerungsglied aus dieser festen Position gelöst werden und relativ zu der Kolbenstange, bzw. dem Antriebsglied, verschoben werden. Soll vor der Überwindung der Leerhubstrecke die Dosis verabreicht werden, ist der Kontakt zwischen Verzögerungsglied und Antriebsglied in der ersten Stellung so fest, dass die Kolbenstange über die Dosisweglänge verschoben wird. Danach schlägt sie z. B. an einem Anschlag an, um ihre Verschiebung zu stoppen, so dass durch weitere Betätigung des Antriebsknopfes der Kontakt zwischen Verzögerungsglied und Antriebsglied gelöst wird und das Verzögerungsglied über die Leerhubstrecke in die zweite Stellung verschoben wird. Der momentane Zustand der Verabreichungsvorrichtung während der Dosisverabreichung kann durch die lösbar festen Stellungen markiert werden, so dass die Stellungen für den Anwender spürbar oder auch hörbar sind.

Erfindungsgemäß wird zwischen dem wenigstens einen Verzögerungsglied und dem Antriebsglied ein Kontakt derart hergestellt, dass bei Betätigung des Antriebsknopfes das Antriebsglied in Vorschubrichtung verschiebbar ist. Der Kontakt wird dabei entweder in der ersten Stellung des Verzögerungsglieds bevor oder in der zweiten Stellung nachdem das Verzögerungsglied relativ zum Antriebsglied verschoben wurde hergestellt. Das heißt mit der Erfindung ist es möglich, einen Leerhub entweder vor oder nach der Dosisverabreichung vorzusehen und dadurch die Betätigungsstrecke des Antriebsknopfes bei einer Verabreichung zu verlängern. Vorzugsweise erfolgt ein Leerhub nach der Verabreichung der Dosis. Dadurch kann eine zuverlässige Ausschüttung der Dosis sichergestellt werden.

Ein Kontakt zwischen dem Verzögerungsglied und der Kolbenstange, bzw. dem Antriebsglied, kann bei der Betätigung des Antriebsknopfes durch ein axiales Aufeinanderstoßen zweier Stoßkanten in Längsrichtung der Vorrichtung hergestellt werden. Dabei ist eine Stoßkante mit einer radial verlaufenden Fläche an dem Verzögerungsglied und eine weitere Stoßkante an der Kolbenstange, deren Fläche bei einem Kontakt auf der Fläche der ersten Stoßkante zu liegen kommt, ausgebildet.

Bei der erfindungsgemäß ausgebildeten Verabreichungsvorrichtung wird bei Betätigung des Antriebsknopfes in Vorschubrichtung zunächst eine Leerhubstrecke überwunden. Dabei wird bei der Bewegung des Betätigungsknopfes und damit auch des Verzögerungsgliedes aus der ersten Stellung in die zweite Stellung der Kolben nicht bewegt und damit auch kein Produkt ausgeschüttet. Erst nachdem das Verzögerungsglied bei der zweiten Stellung den Kontakt mit der Kolbenstange, bzw. dem Antriebsglied, hergestellt hat und der Antriebsknopf weiter betätigt wird, wird auch die Kolbenstange mit dem Kolben in Vorschubrichtung über eine Dosisweglänge vorgeschoben, sodass eine Produktdosis aus dem Reservoir verdrängt wird. Dabei ist es vorteilhaft, wenn der Abstand zwischen der ersten Stellung und der zweiten Stellung entlang der Längsachse der Verabreichungsvorrichtung, d.h. die Leerhubstrecke, beachtlich größer als die Dosisweglänge ist, die zur Dosisabgabe von dem Kolben überwunden wird. Vorzugsweise beträgt die Leerhubstrecke mehrere Male die Dosisweglänge.

Bei der Verabreichung eines Produkts mit der erfindungsgemäßen Vorrichtung wird durch den Leerhub die Gesamtstrecke, in die ein Antriebsknopf vorgeschoben, bzw. in das Gehäuse der Vorrichtung hineingedrückt wird, im Gegensatz zum Stand der Technik deutlich verlängert. Durch diese verlängerte Wegstrecke wird es einem Anwender ermöglicht, seinen Krafteinsatz bei der Betätigung des Antriebsknopfes besser zu kontrollieren, wodurch eine leichtere Führung der Verabreichungsvorrichtung bei der Dosisabgabe möglich ist. Durch einen kontinuierlichen Übergang von der Betätigung zur Überwindung der Leerhubstrecke zur Überwindung der Dosisweglänge oder umgekehrt kann z. B. eine sanfte Injektion eines fluiden Produkts erfolgen. Vor allem bei kleinen Dosisvolumina, d.h. bei kleinen Dosisweglängen ist der Verabreichungsvorgang für einen Anwender erleichtert, da eine verlängerte Betätigungsstrecke anschaulicher ist und die erste und zweite Stellung die Position und den Zustand der Vorrichtung während der Verabreichung wahrnehmbar anzeigen.

Die Dosisweglänge kann bei der Verabreichungsvorrichtung eine für jede Injektion gleichbleibende Weglänge sein. Vorzugsweise ist jedoch an der Verabreichungsvorrichtung eine Dosiereinrichtung vorgesehen. Hierfür kann ein Hülsenkörper ein Dosierglied bilden, das mit einem hinteren Gehäuseteil verschiebegesichert, jedoch um die gemeinsame Längsachse drehbar, verbunden ist. Durch ein Verdrehen dieses Dosierglieds wird die in Vorschubrichtung von der Kolbenstange maximal zurücklegbare Dosisweglänge eingestellt. Das Dosierglied kann beispielsweise entsprechend einem in der WO 97/36625 beschriebenen Dosierglied ausgebildet sein und bei der Dosierung mit einem Antriebsglied wie dort beschrieben zusammenwirken.

In der vorliegenden Erfindung wird die erste und die zweite Stellung durch ein Führungsprofil im Inneren der Verabreichungsvorrichtung bestimmt. Das Führungsprofil wird durch die Ausgestaltung einer Oberfläche gebildet, die dem Verzögerungsglied gegenüberliegt und an der das Verzögerungsglied entlang gleitet. Das Führungsprofil ist auf der Außenmantelfläche des Antriebsglieds oder der Kolbenstange vorgesehen. Das Verzögerungsglied weist wenigstens einen Vorsprung auf, der durch die Betätigung des Antriebsknopfes entlang dem Führungsprofil geführt wird. Vorzugsweise ist der Vorsprung in ständigem Kontakt mit der Profiloberfläche und die zwischen der ersten und der zweiten Stellung zu überwindende Fläche weist einen definierten Reibwiderstand auf. Der Reibwiderstand kann derart ausgebildet sein, dass die Kraft, die zur Verschiebung zwischen den zwei Stellungen des Verzögerungsgliedes notwendig ist, annähernd der Kraft entspricht, die zur Betätigung des Antriebsknopfes zur Ausschüttung des Produkts erforderlich ist. Dadurch entsteht eine kontinuierliche Bewegung vom Beginn der Betätigung des Antriebsknopfes bis zur Vollendung der Dosisausschüttung, bei der durch die erste und zweite Stellung der Zustand während der Verabreichung durch einen vorübergehend höheren Widerstand markiert wird. Zur Ausbildung der Reibflächen können die Bauteile der Verabreichungsvorrichtung als Zweikomponenten-Spritzgussteile hergestellt werden, die eine hart-weich Verbindung haben, so dass Reibflächen mit unterschiedlichen Widerständen entstehen.

In der Erfindung wird das Führungsprofil durch eine Führungsschiene gebildet, die in einer Außenmantelfläche einer Kolbenstange, bzw. eines Antriebsgliedes, in Längsrichtung eingelassen ist. In der Führungsschiene ist eine Vertiefung für die erste Stellung und eine zweite Vertiefung für die zweite Stellung des Verzögerungsglieds ausgebildet, wobei die erste Vertiefung bevorzugt an einem Ende der Führungsschiene und die zweite Vertiefung am anderen Ende ist. An der der Führungsschiene gegenüberliegenden Fläche des Verzögerungsglieds ist ein Vorsprung vorgesehen, der in einer ersten lösbar festen Stellung in die erste Vertiefung auf der Oberfläche des Antriebsgliedes eingreift. Durch die Betätigung des Antriebsknopfes wird der Widerstand überwunden, der durch das Eingreifen des Vorsprungs in die Vertiefung entsteht. Der Widerstand ist größer ausgebildet, wenn zuerst eine Dosisweglänge überwunden werden soll, wozu das Verzögerungsglied beim Vorschub in der ersten Stellung bleibt. Er ist kleiner ausgebildet, wenn zuerst die Leerhubstrecke überwunden wird, um das Verzögerungsglied leicht aus der ersten Stellung lösen zu können. Nach dem Überwinden des Widerstandes wird der Vorsprung entlang der eingelassenen Führungsschiene verschoben, bis er in eine zweite lösbar feste Stellung in der zweiten Vertiefung einrastet. In dieser Stellung kann gleichzeitig ein Kontakt zwischen dem Betätigungsknopf und der Kolbenstange, bzw. einem mit der Kolbenstange verbundenen Antriebsglied hergestellt werden. An einer anderen Stelle der Kolbenstange oder des Antriebsgliedes kann ein zusätzliches Führungsprofil vorgesehen sein, das gleichfalls als eingelassene Führungsschiene ausgebildet ist. Eine Stufe oder ein Absatz an dem Verzögerungsglied kann in diese Führungsschiene eingreifen und als eine zusätzliche Führung bei der Bewegung des Verzögerungsgliedes zwischen den zwei lösbar festen Stellungen wirken. Das Einrasten des Vorsprungs des Verzögerungsgliedes in die Vertiefungen der ersten Führungsschiene ist für einen Anwender spürbar, sodass er eine Rückmeldung über den Vorgang bei der Verabreichung erhält.

Es ist auch möglich, die zweite lösbar feste Stellung des Verzögerungsgliedes zusätzlich durch das Aufeinandertreffen zweier Stoßkanten für einen Kontakt zwischen dem Verzögerungsglied und der Kolbenstange zu definieren. Hierfür kann eine radial verlaufende Stoßkante des Verzögerungsgliedes z.B. gegen die Stirnfläche des Antriebsgliedes stoßen.

In einem anderen Beispiel, welches nicht Teil der beanspruchten Erfindung ist, ist die Kolbenstange als Zahnstange ausgebildet und das Verzögerungsglied weist wenigstens einen Mitnehmer, wie etwa eine Kralle auf, das in eine Zahnreihe der Zahnstange eingreifen kann. Das Verzögerungsmittel ist stegförmig in Verlängerung an einem Antriebsknopf angebracht, sodass es zunächst nicht in die Zahnreihe eingreift. An einer dem Verzögerungsglied gegenüberliegenden Innenmantelfläche des Gehäuses oder eines Dosiergliedes ist ein stufenförmig ausgebildetes Führungsprofil vorgesehen. Die Stufen sind in Vorschubrichtung radial nach innen ansteigend ausgebildet. Das Verzögerungsglied weist auf der dieser Innenmantelfläche zugewandten Seite einen Vorsprung auf, der an der Profiloberfläche anliegt. Zunächst wird das Verzögerungsglied mittels dem Antriebsknopf entlang einer ersten Fläche, die der Leerhubstrecke entspricht, in Vorschubrichtung entlang dem Führungsprofil verschoben bis zu einer ersten Stufe des Führungsprofils. Diese erste Stufe wird bei fortgesetzter Betätigung des Antriebsknopfes von dem Vorsprung überwunden, wodurch das Verzögerungsglied radial in Richtung der Zahnstange gebogen wird. Vorzugsweise ist die Stufenfläche schräg zur Längsachse der Vorrichtung angeordnet, um das Überwinden der Stufe zu erleichtern. Beim Überwinden der Stufe greift die Kralle des Verzögerungsgliedes in die Zahnreihe der Zahnstange ein und stößt gegen eine Zahnflanke der Zahnreihe, wodurch sich ein erfindungsgemäßer Kontakt einstellt. In dieser Stellung befindet sich das Verzögerungsglied in der zweiten Stellung relativ zu der Zahnstange.

Bei fortgesetzter Betätigung des Antriebsknopfes wird der Vorsprung des Verzögerungsgliedes entlang einer zweiten Fläche in dem Führungsprofil, die sich an die erste Stufe anschließt, geführt. Dabei steht das Verzögerungsglied in Eingriff mit der Kolbenstange, bzw. dem Antriebsglied, und verschiebt diese somit in Vorschubrichtung, sodass Produkt aus dem Reservoir verdrängt wird. Diese zweite Fläche in dem Führungsprofil entspricht vorzugsweise der Dosisweglänge für die Produktverabreichung. Nach dem Überschreiten dieser Fläche stößt der Vorsprung vorzugsweise gegen eine zweite Stufe des Führungsprofils, wodurch eine Dosisverabreichung beendet wird. Die zweite Stufe kann auch durch einen Anschlag gebildet werden, der mittels einer Dosiereinrichtung in Vorschubrichtung veränderlich vorgesehen werden kann. Zudem kann es vorteilhaft sein, dass das Verzögerungsglied mit seiner Stirnfläche gegen eine nach innen gerichtete Kante des Gehäuses stößt, die als zusätzliche Sicherung gegen ein weiteres Verschieben der Kolbenstange dienen kann.

Beim Zurückziehen des Antriebsknopfes für eine nachfolgende Verabreichung verhindert eine Rückzugssperre in Form eines Sperrmittels, das in die Zahnreihe der Zahnstange eingreift, dass die Zahnstange ebenfalls zurückgezogen wird. Die Mitnehmer, bzw. Krallen des Verzögerungsgliedes werden über die flachen Flanken der Zahnreihe zurückgezogen, wobei sich das Verzögerungsglied aufgrund seiner Elastizität durch die stegförmige Ausbildung zunächst verbiegt, bis der Vorsprung auf dem Führungsprofil die erste Stufe radial auswärts überwunden hat, so dass das Verzögerungsglied nicht mehr zwischen die Zähne der Zahnreihe eingreift. Das Verzögerungsglied bzw. der Antriebsknopf werden soweit zurückgezogen, bis das Verzögerungsglied wieder in seiner ersten Stellung an einer rückwärtigen dritten Stufe des Führungsprofils zu liegen kommt. Die Verabreichungsvorrichtung ist sodann bereit für eine erneute Verabreichung. Bei jeder Verabreichung wird vorzugsweise die Zahnstange um einen Zahnabstand, welcher der Dosisweglänge entspricht, weiter in Vorschubrichtung geschoben. Eine eigene Dosiereinrichtung ist bei dieser Ausführungsform nicht unbedingt erforderlich.

Bei einer weiteren Ausführungsform der vorliegenden Erfindung ist es möglich, mehrere Verzögerungsglieder vorzusehen, die teleskopartig ineinander geführt werden und jeweils eine erste und eine zweite lösbar feste Stellung relativ zueinander, bzw. zu der Kolbenstange, aufweisen. Bei einer Betätigung des Antriebsknopfes wird dann zuerst eines der Verzögerungsglieder aus seiner ersten Stellung in seine zweite Stellung gebracht, und bei fortgesetzter Betätigung des Antriebsknopfes dann ein zweites Verzögerungsglied aus seiner ersten Stellung in seine zweite Stellung gebracht wird. Sobald das letzte Verzögerungsglied seine zweite Stellung erreicht hat, ist der Kontakt mit der Kolbenstange oder mit einem Antriebsglied für die Kolbenstange hergestellt, sodass eine Dosis verabreicht werden kann.

Bevorzugte Ausführungsformen der Erfindung werden nachfolgend beispielhaft anhand der Zeichnungen 1 und 2 erläutert. In dieser stellen dar:
- Figur 1:: einen Querschnitt in Längsrichtung durch einen Teil einer Verabreichungsvorrichtung mit einer Antriebseinrichtung und einem Verzögerungsglied gemäß einer ersten Ausführungsform der vorliegenden Erfindung,
- Figur 2:: einen Querschnitt durch den Teil der Verabreichungsvorrichtung mit dem erfindungsgemäßen Verzögerungsglied als Vergrößerung aus Figur 1,
- Figur 3:: einen Querschnitt ins Längsrichtung durch einer Verabreichungsvorrichtung, welche nicht Teil der Beanspruchten Erfindung ist, mit einem Verzögerungsglied in einer ersten Stellung,
- Figur 4:: einen Querschnitt nach Figur 3 mit dem Verzögerungsglied beim Eingriff in eine Zahnstange,
- Figur 5:: einen Querschnitt nach Figur 3 mit dem Verzögerungsglied in einer zweiten Stellung.
- Figur 6:: einen Querschnitt durch eine Verabreichungsvorrichtung wie bei Figur 3 nach der letzten verabreichten Dosis,
- Figur 7:: einen Querschnitt durch einen Teil der Verabreichungsvorrichtung mit der Antriebsrichtung in einem entriegelten Zustand mit eingedrücktem Antriebsknopf,
- Figur 8:: einen Querschnitt nach Figur 7 mit einem zurückgezogenen Antriebsknopf und
- Figur 9:: einen Querschnitt wie Figur 7 mit einer zurückgezogenen Zahnstange in einem verriegelten Zustand.

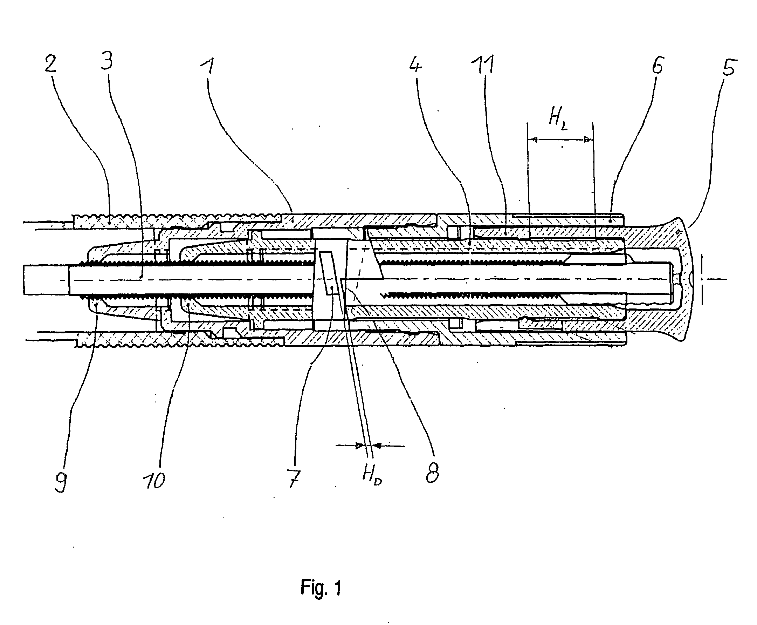

In Figur 1 ist eine Antriebseinrichtung in einem Bereich einer Verabreichungsvorrichtung in Form eines Injektionspens gezeigt. Der Injektionspen weist ein Gehäuse mit einer Gehäusehülse 1 in dem Bereich mit der Antriebseinrichtung und eine Gehäusehülse 2 in einem Bereich auf, der sich an den Verabreichungsmechanismus anschließt und eine Ampulle (nicht gezeigt) mit einem injizierbaren Produkt als Produktreservoir aufnimmt. Das Produkt ist ein fluides Produkt, z.B. eine Wirkstofflösung wie Insulin. In der Ampulle ist ein Kolben aufgenommen, der bei Verschiebung in Vorschubrichtung das Produkt aus der Ampulle durch einen Ampullenauslass verdrängt und durch eine Injektionsnadel ausschüttet. Die Verschiebung des Kolbens in der Ampulle in Vorschubrichtung wird durch die Antriebseinrichtung bewirkt, die als Kolbenstange eine Zahnstange 3, die unmittelbar auf den Kolben wirkt, und ein Antriebsglied 4 umfasst. Das Antriebsglied 4 ist in und gegen die Vorschubrichtung des Kolbens verschiebbar gelagert. Ein Antriebsknopf 5 ragt an dem dem Auslass gegenüberliegenden Ende nach hinten aus dem Injektionspen.

Der in Figur 1 gezeigte Injektionspen ist außerdem mit einer Dosiereinrichtung ausgestattet. Hierfür weist er ein als Hülsenkörper ausgebildetes Dosierglied 6 auf, das mit der Gehäusehülse 1 verschiebegesichert ist und mit dieser um die gemeinsame Längsachse verdrehbar verbunden ist. Durch das Verdrehen des Dosierglieds 6 wird die in Vorschubrichtung von dem Antriebsglied 4 und der Zahnstange 3 für ein bestimmtes Dosisvolumen notwendige Dosisweglänge eingestellt. Bei der Dosierung wird das Dosierglied 6 relativ zur Gehäusehülse 1 verdreht, wobei zwischen einem von einer äußeren Mantelfläche des Antriebsglieds 4 abragender Kragen 7 und einer dem Kragen 7 gegenüberliegenden spiralförmig umlaufend ausgebildeten Stirnfläche 8 des Dosierglieds 6 ein Abstand H_{D} entsprechend einer Dosiswegelänge entsteht.

Um die Dosisweglänge kann das Antriebsglied 4 relativ zur Gehäusehülse 1 und damit auch relativ zum Kolben in der Ampulle gegen die Vorschubrichtung zurückgezogen werden. Bei einem Zurückziehen des Antriebsglieds 4 verbleibt die Zahnstange 3 in ihrer bei dem Dosiervorgang eingenommenen Verschiebelage relativ zum Gehäuse. Sie wird durch an der Gehäusehülse 1 ausgebildete Sperrmittel 9 gegen eine Verschiebung gegen die Vorschubrichtung gesichert. Die Sperrmittel 9 sind in der dargestellten Ausführungsform Rastnocken, die je an einem vorderen Ende einer von der Gehäusehülse 1 abragenden elastischen Zunge ausgebildet sind und radial nach innen auf die Zahnstange 3 zuragen. Die Sperrmittel 9 wirken mit einer ihnen zugewandten Zahnreihe der Zahnstange 3 derart zusammen, dass sie eine Verschiebung der Zahnstange 3 in Vorschubrichtung zulassen und eine Verschiebung gegen die Vorschubrichtung durch formschlüssigen Sperreingriff verhindern.

Die Verschiebung der Zahnstange 3 in Vorschubrichtung wird durch das Antriebsglied 4 bewirkt. Hierfür weist das Antriebsglied in Vorschubrichtung ausgebildete Zungen auf, die an ihren vorderen Enden radial nach innen abragende Mitnehmer 10 tragen. Bei einer Verschiebung des Antriebsglieds 4 in Vorschubrichtung greift einer der Mitnehmer 10 in eine ihm zugewandte Zahnreihe der Zahnstange 3 und bewirkt so die zwangsweise Mitnahme der Zahnstange 3 in Vorschubrichtung. Die Zungen des Antriebsglieds 4 mit den Mitnehmern 10 sind elastisch ausgebildet, sodass bei einer Verschiebung des Antriebsglieds 4 gegen die Vorschubrichtung die Mitnehmer über die Zahnreihen der durch die Sperrmittel 9 gesperrten Zahnstange 3 gleiten.

In Figur 1 ist ein erfindungsgemäßes Verzögerungsglied 11 gezeigt, das einstückig mit dem Antriebsknopf 5 ausgebildet ist. Natürlich ist es auch denkbar, das Verzögerungsglied 11 und den Antriebsknopf 5 als getrennte Bauteile in einer festen Verbindung vorzusehen. In der dargestellten Ausführungsform ist das Verzögerungsglied 11 als Verlängerung des Antriebsknopfs 5 vorgesehen. Die Verlängerung besteht vorzugsweise aus einem oder mehreren Stegen 12, 13. Sie kann aber auch von einer Hülse gebildet werden. Das Verzögerungsglied 11 ist in radialer Richtung zwischen dem Antriebsglied 4 und dem Dosierglied 6 angeordnet. Bei einem Injektionspen ohne Dosiereinrichtung kann das Verzögerungsglied 11 z.B. zwischen einem Antriebsglied 4 und einer Gehäusehülse 1 vorgesehen sein. Auch ist ein Injektionspen ohne ein gesondertes Antriebsglied denkbar, wobei das Verzögerungsglied 11 zwischen der Kolbenstange, bzw. Zahnstange, und einer Gehäusehülse anzuordnen ist.

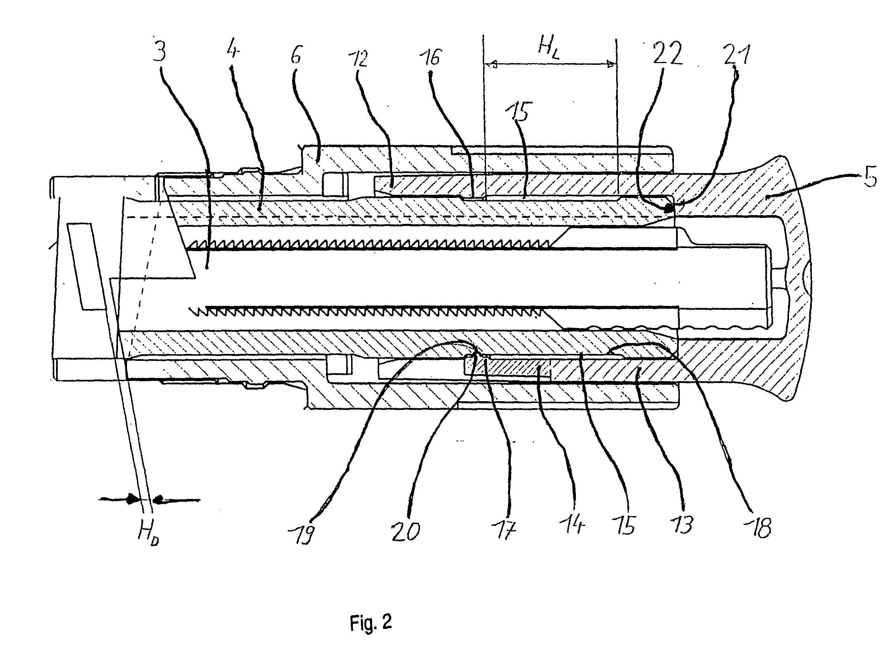

Der Bereich des Injektionspens mit dem Verzögerungsglied 11 ist in Figur 2 vergrößert dargestellt. Das Verzögerungsglied 11 weist einen Steg 12 und einen diesem gegenüberliegend angeordneten Steg 13 auf. Der Steg 13 ist an seiner Stirnfläche mit einer Verlängerung 14 versehen, die eine größere Flexibilität bzw. Biegsamkeit aufweist, als der Steg 13. Auf der Außenmantelfläche des Antriebsglieds 4 sind an den Stegen 12 und 13 gegenüberliegenden Flächen Führungsschienen 15 eingelassen, die sich in Längsrichtung des Injektionspens erstrecken. An den radial nach innen weisenden Flächen des Steges 12 und der Verlängerung 14 ist jeweils eine Stufe 16 für den Steg 12 und eine Stufe 17 für die Verlängerung 14 angeordnet. Die Stufen sind so hoch ausgebildet, dass sie in die Führungsschienen 15 eingreifen, die Außenmantelfläche des Antriebsglieds 4 und die Innenfläche der Stege 12 und 13 aber dennoch aufeinander zu liegen kommen. Die Kanten der Stufen 16 und 17 stoßen demnach an die jeweils gegenüberliegenden Kanten der Führungsschienen 15. Das Verzögerungsglied 11 ist daher im Wesentlichen über die Länge der Führungsschienen 15 relativ zu dem Antriebsglied verschiebbar gelagert.

In der Führungsschiene 15 für die Verlängerung 14 ist in einem dem Antriebsknopf 5 nahen Bereich für eine erste lösbar feste Stellung des Verzögerungsglieds 11 eine erste Vertiefung 18 vorgesehen. In einem vom Antriebsknopf 5 entfernten Bereich der Führungsschiene 15 ist für eine zweite lösbar feste Stellung des Verzögerungsglieds 11 eine zweite Vertiefung 19 ausgebildet. Auf der Stufe 17 der Verlängerung 14 ragt ein Nocken 20 in Richtung der Vertiefungen 18 und 19. Die Verlängerung 14 ist derart flexibel vorgespannt, dass der Nocken 20 in die Vertiefungen 18 und 19 hineinragt, wenn er diesen gegenüber liegt. In einer Stellung zwischen den Vertiefungen 18 und 19 wird die Verlängerung 14 um die Höhe der Vertiefungen zurückgebogen, sodass die Verlängerung 14 bei einem Verschieben des Verzögerungsgliedes 11 gegenüber dem Antriebsglied 4 entlang der Führungsschiene gleitet. Durch das Einrasten des Nockens 20 in die erste und die zweite Vertiefung 18 und 19 werden die erste und die zweite lösbar feste Stellung des Verzögerungsgliedes 11 in Bezug zu dem Antriebsglied 4 für einen Anwender fühlbar und auch hörbar.

Bei der Verschiebung des Verzögerungsgliedes 11 über die Länge der Führungsschiene 15 bleibt die Zahnstange 3 relativ zur Gehäusehülse 1, bzw. zum Dosierglied 6 in Ruhe. Bei einer Betätigung des Antriebsknopfes 5 und damit des Verzögerungsgliedes 11 zwischen der ersten lösbar festen Stellung 18 und der zweiten lösbar festen Stellung 19 wird daher der Kolben in der Ampulle, bzw. dem Reservoir, nicht bewegt, sodass kein Produkt aus dem Reservoir verdrängt wird. Der Abstand zwischen der ersten und der zweiten Vertiefung 18 und 19, bzw. die Länge der Führungsschiene, bilden daher eine Leerhubstrecke H_{L} für die Verabreichungsvorrichtung.

Ist der Nocken 20 in die zweite Vertiefung 19 spürbar eingerastet, d.h. befindet sich das Verzögerungsglied 11 in seiner zweiten lösbar festen Stellung, stößt eine Stirnfläche 21 des Antriebsknopfes 5 gegen eine ihr gegenüberliegende Stirnfläche 22 des Antriebsglieds 4 und stellt einen Kontakt zwischen dem Verzögerungsglied 11 und dem Antriebsglied 4 her. Die Stirnflächen wirken daher als Stoßkanten für den Kontakt. Die Stirnfläche 21 des Antriebsknopfes 5 kann auch als radial nach innen ragende Stufe des Verzögerungsglieds 11 betrachtet werden und bildet daher einen Übergang zwischen dem Verzögerungsglied 1 und dem Dosierknopf 5. Wird der Dosierknopf 5 aus dieser zweiten Stellung weiter in Vorschubrichtung betätigt, dann wird über den Kontakt das Antriebsglied 4 relativ zur Gehäusehülse 1 in Vorschubrichtung verschoben. Der Antriebsknopf 5 kann so lange in Vorschubrichtung verschoben werden, bis die in Figur 1 dargestellte Dosisweglänge H_{D} überwunden ist und das Antriebsglied 4 gegen eine Stoßkante an der Gehäusehülse 1 stößt:

In seiner vollständig herausgezogenen Stellung befindet sich der Antriebsknopf 5 in der ersten lösbar festen Stellung, d.h. der Nocken 20 ragt in die erste Vertiefung 18. Beim Betätigen des Antriebsknopfes in Vorschubrichtung kann sich der Nocken 20 aus der Vertiefung 18 lösen, und die Stufen 16 und 17 gleiten in ihren jeweiligen Führungsschienen 15 über die Leerhubstrecke H_{L}, bis der Nocken 20 in die zweite Vertiefung 19 eingreift, sodass das Verzögerungsglied 11 in einer zweiten lösbar festen Stellung ist. In dieser zweiten Stellung wird der Kontakt zwischen dem Verzögerungsglied 11, bzw. dem Antriebsknopf 5, und dem Antriebsglied 4 durch das Aufeinanderstoßen der Stirnfläche 21 des Antriebsknopfes 5 und der Stirnfläche 22 des Antriebsglieds 4 hergestellt. Die fortgesetzte Betätigung des Antriebsknopfes in Vorschubrichtung bewirkt eine Verschiebung des Antriebsglieds 4 relativ zur Gehäusehülse 1 bis die Dosisweglänge H_{D} überwunden ist. Wie ebenfalls der Figur 1 zu entnehmen ist, ist die Leerhubstrecke H_{L} deutlich länger als die Dosisweglänge H_{D}. Dadurch wird es für einen Anwender möglich, bei der Injektion einer kleinen Dosismenge, d.h. bei einer Injektion mit einer nur geringen Dosisweglänge H_{D}, die Verabreichung des Produkts gefühlvoller und mit mehr Sicherheit durchzuführen.

Alternativ ist es möglich, den Widerstand, den der Nocken 20 beim Austreten aus der ersten Vertiefung 18 erfährt, so groß auszubilden, dass bei der Betätigung. des Antriebsknopfes 5 zuerst die Zahnstange 3 vorgeschoben wird, bis die Stirnfläche 8 gegen den Kragen 7 stößt, so dass die Dosisweglänge H_{D} überschritten wurde. Erst dann wird der Widerstand überwunden und die Leerhubstrecke H_{L} zurückgelegt.

Ist das Verzögerungsglied 11 hülsenförmig um das Antriebsglied 4 umlaufend ausgebildet, können auch die Führungsschienen, als ein ringförmiger Bereich des Antriebsglieds 4 mit einem erweiterten Innenumfang ausgebildet sein. Auch die erste Vertiefung 18 und die zweite Vertiefung 19 können dann als ringförmig umlaufende Vertiefungen vorgesehen sein. Zur Bestimmung des Widerstands kann die Breite der Nocken variiert werden. Es können auch mehrere Nocken vorgesehen werden.

Zur Vorbereitung einer nachfolgenden Verabreichung wird der Antriebsknopf 5 entgegen der Vorschubrichtung in eine aus dem Injektionspen herausgezogene Stellung gebracht. Dabei wird zunächst das Verzögerungsglied 11 von der zweiten Stellung in die erste Stellung zurückversetzt und anschließend durch weiteres Herausziehen des Antriebsknopfes 5 das Antriebsglied 4 relativ zur Gehäusehülse 1 entgegen der Vorschubrichtung zurückgezogen. Beim Zurückziehen des Antriebsglieds 4 werden die Mitnehmer 10 an den Zungen des Antriebsglieds 4 über die Zahnreihe der Zahnstange 3 zurückgezogen. Nun kann mit dem Dosierglied 6 eine neue Dosis eingestellt werden, sodass der Injektionspen für eine erneute Verabreichung des injizierbaren Produkts bereit ist.

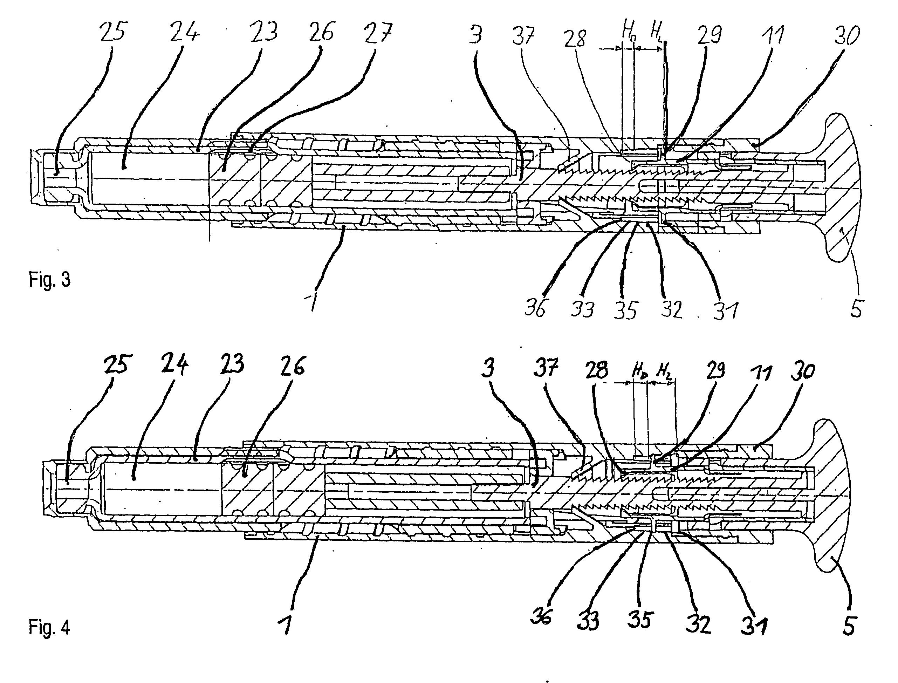

Die Figuren 3 bis 9 zeigen eine Verabreichungsvorrichtung, welche nicht Teil der beanspruchten Erfindung ist. In Figur 3 ist ein Injektionspen mit einer Gehäusehülse 1 dargestellt, in deren vorderem Ende eine Ampulle 23 und deren hinterem Ende eine Antriebseinrichtung aufgenommen ist. Im vorderen Teil der Ampulle 23 ist ein Raum 24 für ein injizierbares Produkt angeordnet, der in einen Auslass 25 mündet. Im hinteren Bereich der Ampulle ist ein Kolben 26 mit einer Verbindung zu einer als Zahnstange ausgebildeten Kolbenstange 3 aufgenommen. Die gezeigte Ausführungsform des Injektionspens weist zudem eine Abmischeinrichtung 27 auf, durch die es möglich ist, ein Zweikomponentenprodukt direkt vor der Verabreichung abzumischen.
Die Antriebseinrichtung im hinteren Teil der Gehäusehülse 1 umfasst einen Antriebsknopf 5, die Zahnstange 3 und ein Verzögerungsglied 11. Der Antriebsknopf 5 und das Verzögerungsglied 11 sind getrennte Bauteile, die fest miteinander verbunden sind. Sie können jedoch auch in dieser Ausführungsform als einstückiges Bauteil vorgesehen werden. Das Verzögerungsglied 11 ist als langgestreckter Steg ausgebildet, der an seinem hinteren Ende an dem Antriebsknopf 5 angebracht ist und an seinem vorderen Ende eine radial nach innen weisende Kralle 28 aufweist. In einem mittleren Bereich weist das Verzögerungsglied 11 einen radial nach außen weisenden Vorsprung 29 auf. In die Gehäusehülse 1 ist eine weitere Gehäusehülse 30 eingesetzt, die mit der Gehäusehülse 1 zusammenwirkt.

In dem in Figur 3 gezeigten Zustand des Injektionspens befindet sich der Antriebsknopf 5 in einem hinteren Anschlag und das Verzögerungsglied 11 in einer ersten Stellung. In dieser ersten Stellung stößt der Vorsprung 29 des Verzögerungsglieds 11 gegen eine radial nach innen weisende Stufe 31 der Gehäusehülse 30. Um eine lösbar feste Stellung zu schaffen weist eine Fläche 32 im Bereich vor der Stufe 31 eine zusätzliche Absenkung auf, in der der Vorsprung 29 aufgrund seiner Biegeelastizität zu liegen kommt. Die Kralle 28 des Verzögerungsglieds 11 ist gegenüber einer Zahnreihe der Zahnstange 3 angeordnet. In der ersten lösbar festen Stellung des Verzögerungsglieds 11 befindet sich die Kralle 28 außer Eingriff mit der Zahnreihe.

Auf der Innenmantelfläche des Gehäuses, die zusammen von der Gehäusehülse 1 und der Gehäusehülse 30 gebildet wird, ist ein stufenförmiges Führungsprofil ausgebildet, dessen Stufen in Vorschubrichtung des Kolbens 26 auf den Auslass 25 zu radial nach innen ansteigen. Das Führungsprofil kann dabei als in Längsrichtung verlaufende stufenförmige Führungsrillen oder als stufenförmig vertiefte Ringbereiche in der Innenmantelfläche des Gehäuses ausgebildet sein. Das Führungsprofil weist eine Fläche 32 und eine Fläche 33 auf, wobei die Fläche 32 ausgehend von der Innenmantelfläche des Gehäuses tiefer in das Gehäuse eingelassen ist als die Fläche 33. Das Führungsprofil umfasst zudem die Stufe 31 im hinteren Bereich der Fläche 32, eine Stufe 35 zwischen der Fläche 32 und der Fläche 33 und eine Stufe 36 im vorderen Bereich der Fläche 33 auf.

An der Innenmantelfläche der Gehäusehülse 1 sind schräg nach innen in Richtung des Auslasses 25 gerichtete Zungen 37 angeordnet. Die Zungen 37 greifen zwischen die Zähne der Zahnreihe der Zahnstange 3, sodass sie eine Verschiebung entgegen der Vorschubrichtung der Zahnstange verhindern und bei einer Verschiebung in Vorschubrichtung flexibel radial nach außen gebogen werden und über die schrägen Flanken der Zahnreihe rutschen.

In dem in Figur 4 gezeigten Zustand des Injektionspens wurde der Antriebsknopf 5 mit dem Verzögerungsglied 11 in Vorschubrichtung auf den Auslass 25 zu verschoben. Dabei gleitet der Vorsprung 29 entlang der Fläche 32 des Führungsprofils von der ersten lösbar festen Stellung aus der Absenkung vor der Stufe 31 in einen vorderen Bereich der Fläche 32, bis er an der Stufe 35 anschlägt. Durch das Überwinden der Absenkung wird das Verzögerungsglied 11 geringfügig radial nach innen gebogen und gerät dadurch unter Vorspannung. Der Anschlag des Vorsprungs 29 an der Stufe 35 bildet eine zweite Stellung für das Verzögerungsglied 11. Auch vor der Kante 35 kann in der Fläche 32 eine Absenkung vorgesehen sein. Die Kralle 28 des Verzögerungsglieds 11 bewegt sich durch die Überwindung der Absenkung für die erste Stellung des Verzögerungsglieds 11 geringfügig in Richtung der Zahnstange 3, greift jedoch nicht in die Zwischenräume der Zahnreihe.

Bei der Bewegung in Vorschubrichtung von der ersten Stellung in die zweite Stellung des Verzögerungsglieds bewegt sich dieses relativ zu der als Kolbenstange wirkende Zahnstange 3 und relativ zu der Gehäusehülse 1. Die Zahnstange 3 und damit der Kolben 26 bleiben dabei relativ zur Gehäusehülse 1 in Ruhe und es wird kein Produkt aus dem Raum 24 verdrängt. Die Bewegung des Antriebsknopfes 5 und des Verzögerungsglieds 11 in Vorschubrichtung entlang der Fläche 32 bildet daher einen Leerhub. Die Leerhubstrecke H_{L} entspricht der Länge der Fläche 32 in Längsrichtung von der Stufe 31 bis zu der Stufe 35. In der gezeigten Ausführungsform beträgt die Leerhubstrecke H_{L} z.B. 6,8 mm.

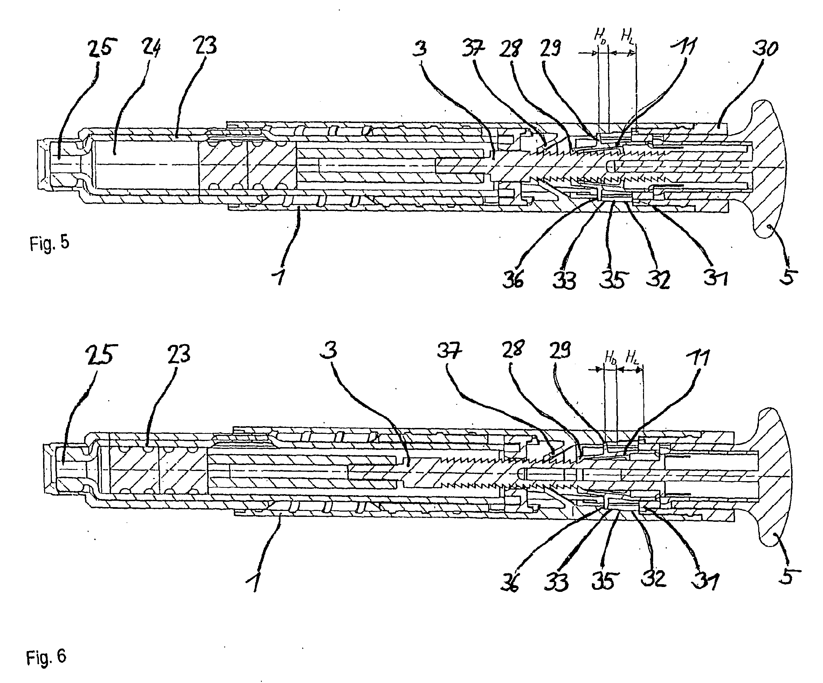

In dem in Figur 5 gezeigten Zustand des Injektionspens ist der Antriebsknopf 5 und damit das Verzögerungsglied 11 weiter in Vorschubrichtung auf den Auslass 25 zu verschoben. Bei diesem Vorschub überwindet der Vorsprung 29 des Verzögerungsglieds 11 bei der zweiten Stellung die Stufe 35, die hierfür geringfügig schräg nach innen in Vorschubrichtung ausgebildet sein kann. Dabei tritt das Verzögerungsglied 11 in Kontakt mit der Zahnstange 3, indem die Kralle 28 zwischen den Zähnen der Zahnreihe der Zahnstange 3 zu liegen kommt und mit ihrer Stirnfläche gegen eine ihr gegenüberliegende senkrecht verlaufende Zahnflanke eines Zahnes der Zahnreihe stößt.

Durch das fortgesetzte Betätigen des Antriebsknopfes 5 wird der Vorsprung 29 entlang der Fläche 33 des Führungsprofils bis zum Anschlag an der Stufe 36 verschoben. Durch den Eingriff der Kralle 28 in die Zahnstange 3 wird diese in Vorschubrichtung mitgenommen, sodass sich der Kolben 26 auf den Auslass 25 zu bewegt und das Produkt aus dem Raum 24 verdrängt. Die Zunge 37 der Gehäusehülse 1 wird bei diesem Vorschub über die ansteigende Flanke eines Zahns der Zahnreihe der Zahnstange 3 von einem Zwischenraum in den nachfolgenden Zwischenraum versetzt. In diesen nachfolgenden Zwischenraum greift sie wieder formschlüssig ein und verhindert ein Verschieben der Zahnstange entgegen der Vorschubrichtung. Die Länge der Fläche 33 von der zweiten Stellung an der Stufe 35 bis zum Anschlag des Vorsprungs 29 an der Stufe 36 entspricht einer Dosisweglänge H_{D} und beträgt in dem gezeigten Ausführungsbeispiel 1,22 mm. Dieser Abstand entspricht auch den Zwischenräumen, bzw. den Zähnen, der Zahnreihe der Zahnstange 3. Bei einer Dosisabgabe wandert daher die Zunge 37 um einen Zahn auf der Zahnreihe der Zahnstange weiter, wobei die Zahnstange 3 relativ zur Gehäusehülse 1 verschoben wird. Gleichzeitig kann eine vordere Stirnfläche des Antriebsknopfs 5 gegen einen Anschlag am Gehäuse stoßen, der außerhalb der Schnittfläche der Figur 5 liegt und daher nicht gezeigt ist.

In Figur 6 ist der Injektionspen nach der Verabreichung mehrerer Dosiseinheiten dargestellt, wobei sich der Kolben 26 in einer vorderen Position innerhalb der Ampulle 23 befindet und die Zungen 37 der Gehäusehülse 1 mehrere Zähne der Zahnreihe der Zahnstange 3 überschritten haben. Das Verzögerungsglied 11 und der Antriebsknopf 5 befinden sich in einer Eingriffsposition vergleichbar mit Figur 5.

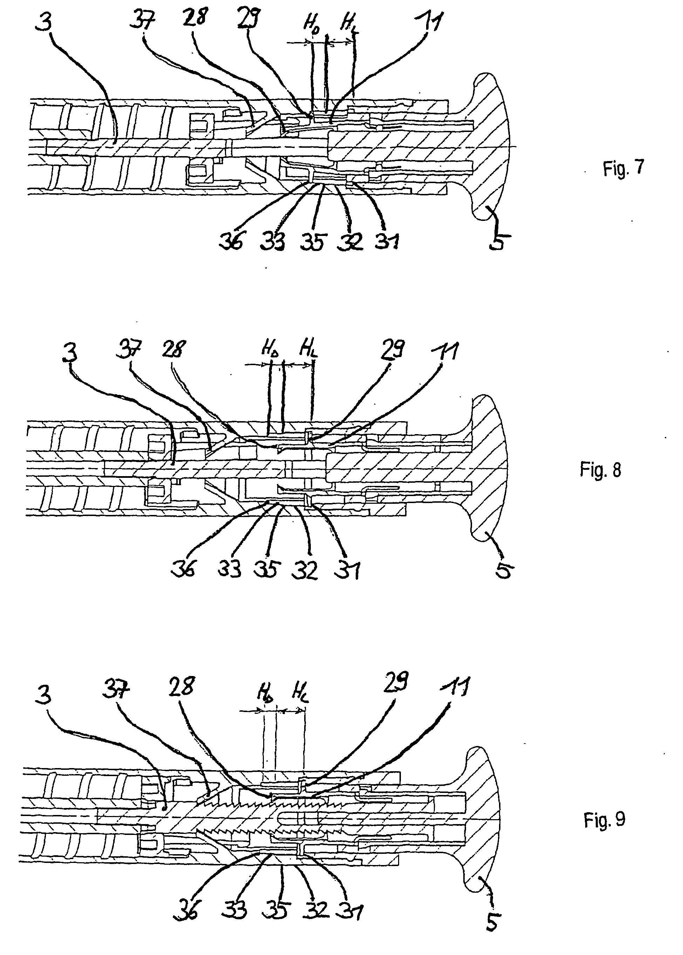

Es ist nun erforderlich, eine neue Ampulle 23 in den Injektionspen einzusetzen und die Antriebseinrichtung in eine Ausgangsposition zurückzuversetzen. Hierfür ist es erforderlich, die Zahnstange 3 entgegen der Vorschubrichtung zurückzuziehen. Wie in Figur 7 gezeigt ist, kann hierfür der Antriebsknopf in Umfangsrichtung des Injektionspens verdreht werden, wobei er die Zahnstange 3 mitnimmt und ebenfalls verdreht. Durch diese Drehung gelangen die Zungen 37 und die Krallen 28 außer Eingriff mit der Zahnstange 3, da die Zahnreihe mit der Zahnstange 3 verdreht wurde und die Zahnstange 3 in dieser verdrehten Position keine Zähne gegenüber den Zungen 37 und den Krallen 28 aufweist. Die Zahnstange 3 befindet sich daher in einer entriegelten Stellung.

In Figur 8 ist der Antriebsknopf 5 und das Verzögerungsglied 11 bis zu dem Anschlag an der Stufe 31 in einer ersten lösbar festen Stellung des Verzögerungsglieds 11 zurückgezogen. Die Zahnstange 3 kann z. B. durch ein Vorspannelement zwischen der Zahnstange 3 und z.B. der Gehäusehülse 1, das durch den Vorschub der Zahnstange 3 in Vorschubrichtung gespannt wird, beim Entriegeln in ihre hintere Position zurückversetzt werden.

Ist die Zahnstange in der hinteren Position, wird der Antriebsknopf 5 und damit die Zahnstange 3 in die ursprünglich Stellung zurückverdreht, wodurch die Zungen 37 in Eingriff mit den Zahnreihen der Zahnstange 3 geraten. Die Antriebseinrichtung des Injektionspens ist damit in einer Ausgangsstellung, in der die Zungen 37 in der ersten Lücke der Zahnreihe der Zahnstange 3 formschlüssig zu liegen kommen. In dem vorderen Bereich der Gehäusehülse 1 kann nun eine neue Ampulle 23 eingesetzt werden. Der Injektionspen ist nun für die weitere Verabreichung eines injizierbaren Produkts bereit.

Der Injektionspen des in den Figuren 3 bis 9 beschriebenen Beispiels weist keine gesonderte Dosiereinrichtung auf. Die Dosis wird durch die Dosisweglänge L_{D}, d.h. durch die Länge der Stufe 33, bzw. den Abstand zwischen den Zähnen der Zahnreihe der Zahnstange 3, bestimmt und entspricht einem Primen. Es wäre jedoch auch denkbar, bei einem Injektionspen mit einem stufenförmig ausgebildeten Führungsprofil eine eigene Dosiereinrichtung vorzusehen, wie es z.B. in der in den Figuren 1 und 2 gezeigten Ausführungsform beschrieben ist.

Durch die Betätigung des Antriebsknopfes 5 durch einen Anwender wird das Verzögerungsglied 11 von seiner ersten lösbar festen Stellung an der Stufe 31 zu seiner zweiten Stellung an der Stufe 35 entlang der Leerhubstrecke H_{L} verschoben. Die erste und die zweite Stellung des Verzögerungsgliedes sind dabei derart beabstandet in dem Führungsprofil angeordnet, dass die Leerhubstrecke H_{L} deutlich größer ist als eine Dosisweglänge H_{D}. In der Ausführungsform der Figuren 3 bis 9 ist die Leerhubstrecke L_{H} mehrmals länger als die Dosisweglänge H_{D}. Durch eine weitere Betätigung des Antriebsknopfes 5 gelangt das Verzögerungsglied 11 bei der zweiten Stellung in Kontakt mit der Zahnstange 3, sodass auch die Dosisweglänge H_{D} von dem Antriebsknopf 5 überwunden wird und die Zahnstange in Vorschubrichtung bewegt wird.

Die Gesamtstrecke, die der Antriebsknopf 5 überwindet, besteht daher aus der Leerhubstrecke H_{L} und aus der Dosisweglänge H_{D}. Für einen Anwender wird es durch diese erweiterte Verabreichungsbewegung des Antriebsknopfes 5 erleichtert, den Ablauf bei der Injektion zu verfolgen und eine sichere Verabreichung des Produkts durchzuführen. Durch das Überwinden der Zunge 37 eines Zahns der Zahnreihe der Zahnstange 3 bei der Dosisverabreichung entsteht ein Klickgeräusch, wenn die Zunge 37 auf die Flanke eines nachfolgenden Zahnes auftrifft. Dadurch kann der Anwender die Verabreichung der Produktdosis auch hörbar wahrnehmen. Durch die Absenkungen in dem Führungsprofil kann er den Zustand des Injektionspens bei der Betätigung des Antriebsknopfes 5 erfühlen, da in den verschiedenen Positionen des Führungsprofils geringfügig unterschiedlicher Druck erforderlich ist. Um den erforderlichen Druck zu definieren und eine kontinuierliche Betätigung des Antriebsknopfes 5 und damit des Verzögerungsglieds 11 herzustellen, können die Flächen 32 und 33 des Führungsprofils als Reibflächen ausgebildet sein, durch die auf den Vorsprung 29 ein gewünschter Reibwiderstand bei der Verschiebung ausgeübt wird. Die Flexibilität des Verzögerungsglieds 11, d.h. dessen Biegesteifigkeit, kann ebenfalls derart ausgewählt werden, dass ein gewünschter Betätigungsverlauf bei der Verabreichung erreicht wird. In der Ausführungsform der Figuren 3 bis 9 wirkt das Verzögerungsglied 11 gleichzeitig als Antriebsglied für die Verabreichung des Produkts.

Die vorliegende Erfindung wurde anhand eines bevorzugten Ausführungsbeispiels beschrieben. Es sind jedoch bei Vorrichtungen zur dosierten Verabreichung eines Produkts auch andere Konstruktionen denkbar, ohne von der Idee der vorliegenden Erfindung abzuweichen. Insbesondere ist es möglich mehrere Gehäuseteile, Antriebsglieder, Verzögerungsglieder, Kolbenstangenelemente und unterschiedliche Sperrmechanismen vorzusehen.

### Bezugszeichen

- 1: Gehäusehülse
- 2: Gehäusehülse
- 3: Zahnstange
- 4: Antriebsglied
- 5: Antriebsknopf
- 6: Dosierglied
- 7: Kragen
- 8: Stirnfläche
- 9: Sperrmittel
- 10: Mitnehmer
- 11: Verzögerungsglied
- 12: Steg
- 13: Steg
- 14: Verlängerung
- 15: Führungsschiene
- 16: Stufe
- 17: Stufe
- 18: erste Vertiefung
- 19: zweite Vertiefung
- 20: Nocken
- 21: Stirnfläche
- 22: Stirnfläche
- 23: Ampulle
- 24: Produktraum
- 25: Auslass
- 26: Kolben
- 27: Abmischeinrichtung
- 28: Kralle
- 29: Vorsprung
- 30: Gehäusehülse
- 31: Stufe
- 32: Fläche
- 33: Fläche
- 34: -
- 35: Stufe
- 36: Stufe
- 37: Zunge
- H_{L}: Leerhubstrecke
- H_{D}: Dosisweglänge

## Patentansprüche

1. Vorrichtung zur dosierten Verabreichung eines injizierbaren Produkts, die umfasst:
a) ein Gehäuse (1; 2) mit einem Reservoir (23) für das Produkt,
b) wenigstens ein Antriebsglied (3, 4) mit einem Kolben (26), das bei einer Verschiebung in Vorschubrichtung mittels des Kolbens (26) das Produkt aus dem Reservoir (23) durch einen Auslass (25) verdrängt,
c) einen Antriebsknopf (5), der bei seiner Betätigung in Vorschubrichtung um eine Betätigungsstrecke relativ zum Gehäuse (1; 2) verschiebbar ist, wobei die Betätigungsstrecke aus einer Dosisweglängen (H_{D}) und einer Leerhubweglänge (H_{L}) besteht, wobei während der Dosisweglänge (L_{D}) der Antriebsknopf (5) das Antriebsglied (3, 4) relativ zum Gehäuse (1; 2) verschiebt und wobei während der Leerhubweglänge (H_{D})
d) wenigstens ein Verzögerungsglied (11), das aus einer ersten Stellung in eine zweite Stellung, die zur ersten Stellung beabstandet ist, durch Betätigung des Antriebsknopfes (5) relativ zu dem Antriebsglied (3, 4) in Längsrichtung der Vorrichtung verschiebbar ist, wobei das Antriebsglied (3, 4) relativ zum Gehäuse (1; 2) in Ruhe bleibt, und
e) das wenigstens eine Verzögerungsglied (11) bei der ersten oder zweiten Stellung einen Kontakt mit dem Antriebsglied (3, 4) derart herstellt, dass bei Betätigung des Antriebsknopfes (5) das Antriebsglied (3, 4) in Vorschubrichtung verschiebbar ist, bevor oder nachdem das wenigstens eine Verzögerungsglied (11) relativ zu dem Antriebsglied (3, 4) verschoben wurde, **dadurch gekennzeichnet, dass**
f) die erste Stellung und die zweite Stellung durch das Zusammenwirken einer ersten und einer zweiten Vertiefung und einem Vorsprung, der an dem Verzögerungsglied (11) angeordnet ist, definiert sind, wobei die erste und die zweite Vertiefung in einer Führungsschiene ausgebildet sind, und die Führungsschiene an einer dem Verzögerungsglied (11) gegenüberliegenden Außenmantelfläche des Antriebsglieds (3,4) in Längsrichtung eingelassen ist wobei der Vorsprung entlang der Führungsschiene verschoben werden kann.

2. Verabreichungsvorrichtung nach Anspruch 1, **dadurch gekennzeichnet, dass** das Verzögerungsglied (11) einstückig mit dem Antriebsknopf (5) ausgebildet ist.

3. Verabreichungsvorrichtung nach Anspruch 1 oder 2, **dadurch gekennzeichnet, dass** die erste Stellung und die zweite Stellung des Verzögerungsglieds (11) lösbar feste Stellungen sind.

4. Verabreichungsvorrichtung nach einem der vorhergehenden Ansprüche, **dadurch gekennzeichnet, dass** der Abstand zwischen der ersten Stellung und der zweiten Stellung des Verzögerungsglieds (11) entlang der Längsachse der Vorrichtung eine Leerhubstrecke (L_{H}) bildet, die mehrfach länger ist als die Dosisweglänge (L_{D}), die von der Kolbenstange zur Verabreichung einer Produktdosis in Vorschubrichtung zurückgelegt wird.

5. Verabreichungsvorrichtung nach einem der vorhergehenden Ansprüche, **dadurch gekennzeichnet, dass** ferner die zweite Position durch das Zusammenstossen einer Stirnfläche (21) des Antriebsknopfs (5) gegen eine ihr gegenüberliegende Stirnfläche (22) des Antriebsgliedes (4) definiert ist.

6. Verabreichungsvorrichtung nach einem der vorhergehenden Ansprüche, **dadurch gekennzeichnet, dass** wenigstens zwei Verzögerungsglieder (11) teleskopartig bei Betätigung des Antriebsknopfes (5) in Längsrichtung der Vorrichtung gegeneinander verschiebbar sind.

7. Verabreichungsvorrichtung nach einem der vorhergehenden Ansprüche, **dadurch gekennzeichnet, dass** wenigstens eine Reibfläche zwischen dem Verzögerungsglied (11) und dem Antriebsglieds (3, 4) einen definierten Reibwiderstand aufweist.

## Claims

1. A device for the dosed administration of an injectable product, which includes:
a) a housing (1; 2) with a reservoir (23) for the product,
b) at least one drive member (3, 4) with a piston (26), which displaces the product out of the reservoir (23) through an outlet (25) upon a displacement in the advance direction by means of the piston (26),
c) a drive knob (5) which is displaceable by an actuating distance relative to the housing (1; 2) upon its actuation in the advance direction, wherein the actuating distance comprises a dose travel length (H_{D}) and an idle stroke travel length (H_{L}), wherein during the dose travel length (H_{D}) the drive knob (5) displaces the drive member (3, 4) relative to the housing (1; 2) and wherein during the idle stroke travel length (H_{L})
d) at least one delay member (11) which is displaceable from a first position into a second position spaced relative to the first position by actuation of the drive knob (5) relative to the drive member (3, 4) in the longitudinal direction of the device, wherein the drive member (3, 4) remains at rest relative to the housing (1; 2), and
e) the at least one delay member (11) in the first or second position makes contact with the drive member (3, 4) in such a way that upon actuation of the drive knob (5) the drive member (3, 4) is displaceable in the advance direction before or after the at least one delay member (11) has been displaced relative to the drive member (3, 4),
**characterised in that**
f) the first position and the second position are defined by the cooperation of a first and a second recess and a projection arranged on the delay member (11), wherein the first and second recess are provided in a guide rail and the guide rail is let in at an outer peripheral surface of the drive member (3, 4), that is in opposite relationship to the delay member (11), in the longitudinal direction, wherein the projection can be displaced along the guide rail.

2. An administration device according to claim 1 **characterised in that** the delay member (11) is integrally formed with the drive knob (5).

3. An administration device according to claim 1 or claim 2 **characterised in that** the first position and the second position of the delay member (11) are releasably fixed positions.

4. An administration device according to one of the preceding claims **characterised in that** the spacing between the first position and the second position of the delay member (11) along the longitudinal axis of the device forms an idle stroke distance (H_{L}) which is a multiple longer than the dose travel length (H_{D}) which is covered by the piston rod for administration of a product dose in the advance direction.

5. An administration device according to one of the preceding claims **characterised in that** in addition the second position is defined by an end face (21) of the drive knob (5) colliding against an end face (22) opposite thereto of the drive member (4).

6. An administration device according to one of the preceding claims **characterised in that** at least two delay members (11) are displaceable relative to each other telescopically upon actuation of the drive knob (5) in the longitudinal direction of the device.

7. An administration device according to one of the preceding claims **characterised in that** at least one friction surface between the delay member (11) and the drive member (3, 4) has a defined frictional resistance.

## Revendications

1. Dispositif d'administration dosée d'un produit injectable, qui comprend :
a) un boîtier (1, 2) doté d'un réservoir (23) pour le produit,
b) au moins un élément d'entraînement (3, 4) doté d'un piston (26) qui déplace le produit hors du réservoir (23) à travers une sortie (25) en cas de poussée dans le sens de l'avance au moyen du piston (26),
c) un bouton de commande (5) qui, lorsqu'il est actionné dans le sens de l'avance, peut être coulissé sur une course d'actionnement par rapport au boîtier (1, 2), moyennant quoi la course d'actionnement se compose d'une longueur de dosage (H_{O}) et d'une longueur à vide (H_{L}), moyennant quoi, pendant la longueur de dosage (H_{O}), le bouton de commande (5) déplace l'élément d'entraînement (3, 4) par rapport au boîtier (1, 2) et moyennant quoi, pendant la course à vide (H_{L})
d) au moins un dispositif de retard (11), qui peut être déplacé d'une première position vers une deuxième position, qui est à distance de la première position, via l'actionnement du bouton de commande (5) par rapport à l'élément d'entraînement (3, 4) dans le sens de la longueur du dispositif, moyennant quoi l'élément d'entraînement (3, 4) reste au repos par rapport au boîtier (1, 2), et
e) le au moins un dispositif de retard (11) établit un contact avec l'élément d'entraînement (3, 4) en première ou deuxième position de telle sorte que, en cas d'actionnement du bouton de commande (5), l'élément d'entraînement (3, 4) peut être déplacé dans le sens de l'avance, avant ou après que le au moins un dispositif de retard (11) a été déplacé par rapport à l'élément d'entraînement (3, 4),
**caractérisé en ce que**
f) la première position et la deuxième position sont définies par l'action conjointe d'un premier et d'un deuxième creux et d'une saillie, qui est disposée sur le dispositif de retard (11), moyennant quoi le premier et le deuxième creux sont formés dans un rail de guidage et le rail de guidage est enchâssé sur une surface d'enveloppe extérieure de l'élément d'entraînement (3, 4) faisant face au dispositif de retard (11) dans le sens longitudinal, moyennant quoi la saillie peut être déplacée le long du rail de guidage.

2. Dispositif d'administration selon la revendication 1, **caractérisé en ce que** le dispositif de retard (11) est conçu d'un seul tenant avec le bouton de commande (5).

3. Dispositif d'administration selon la revendication 1 ou 2, **caractérisé en ce que** la première position et la deuxième position du dispositif de retard (11) sont des positions fixes détachables.

4. Dispositif d'administration selon l'une des revendications précédentes, **caractérisé en ce que** l'écart entre la première position et la deuxième position du dispositif de retard (11) forme une longueur à vide (L_{H}) le long de l'axe longitudinal du dispositif, laquelle longueur à vide est plusieurs fois plus longue que la longueur de dosage (L_{D}) qui est effectuée par la tige du piston pour administrer une dose de produit dans le sens de l'avance.

5. Dispositif d'administration selon l'une des revendications précédentes, **caractérisé en ce que** la deuxième position est entre outre définie par la collision d'une surface de contact (21) du bouton de commande (5) contre une surface de contact (21) faisant face à celle-ci de l'élément d'entraînement (4).

6. Dispositif d'administration selon l'une des revendications précédentes, **caractérisé en ce qu'**au moins deux dispositifs de retard (11) peuvent être déplacés l'un contre l'autre de manière télescopique via l'actionnement du bouton de commande (5) dans le sens longitudinal du dispositif.

7. Dispositif d'administration selon l'une des revendications précédentes, **caractérisé en ce qu'**au moins une surface de friction entre le dispositif de retard (11) et l'élément d'entraînement (3, 4) comprend une résistance à la friction définie.
